(19) Europäisches Patentamt
European Patent Office
Office européen des brevets

(11) Publication number: 0 173 384 B1

(12) # EUROPEAN PATENT SPECIFICATION

(45) Date of publication of patent specification: 16.01.91

(51) Int. Cl.[5]: **C 07 K 7/06**, C 07 K 7/08 // A61K37/02, G01N33/68

(21) Application number: 85201279.8

(22) Date of filing: 07.08.85

(54) **Polypeptide relating to alpha-hANP.**

(30) Priority: 24.08.84 JP 176953/84

(43) Date of publication of application: 05.03.86 Bulletin 86/10

(45) Publication of the grant of the patent: 16.01.91 Bulletin 91/03

(84) Designated Contracting States: BE CH DE FR IT LI NL SE

(56) References cited:
EP-A-0 140 731
EP-A-0 147 193
EP-A-0 164 273
EP-A-0 166 585
WO-A-85/02850

CHEMICAL ABSTRACTS, vol. 103, 1985, page 720, abstract no. 178620k, Columbus, Ohio, US; R.F. NUTT et al.: "Synthesis of peptides with atrial natriuretic factor sequence", & PEPT., PROC. EUR. PEPT. SYMP., 18th 1984, 513-16

(73) Proprietor: SHIONOGI & CO., LTD.
1-8, Doshomachi 3-chome Chuo-ku
Osaka 541 (JP)

(72) Inventor: Kiso, Yoshiaki
15-26, Inaba-cho
Ibaraki-shi Osaka (JP)
Inventor: Nakao, Kazuwa
2-24-6, Oekitafukunishi-cho Nishikyo-ku
Kyoto-shi Kyoto (JP)

(74) Representative: Bruin, Cornelis Willem et al
Octrooibureau Arnold & Siedsma Isartorplatz 5
D-8000 München 2 (DE)

(56) References cited:
CHEMICAL ABSTRACTS, vol. 103, 1985, page 95, abstract no. 172233q, Columbus, Ohio, US; F. BEX et al.: "Atrial natriuretic factor stimulates testosterone production by mouse interstitial cells", & EUR. J. PHARMACOL. 1985, 115(1), 125-6

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 173 384 B1

(56) References cited:

CHEMICAL ABSTRACTS, vol. 104, 1986, page 82, abstract no. 951b, Columbus, Ohio, US; K. INUI et al.: "Specific binding activities and cyclic GMP responses by atrial natriuretic polypeptide in kidney epithelial cell line (LLC-PK1)", & BIOCHEM. BIOPHYS. RES. COMMUN. 1985, 132(1), 253-60

CHEMICAL ABSTRACTS, vol. 104, 1986, page 81, abstract no. 82169q, Columbus, Ohio, US; B. BALLERMANN et al.: "Physiologic regulation of atrial natriuretic peptide receptors in rat renal glomeruli", & J. CLIN. INVEST. 1985, 76(6), 2049-56

CHEMICAL ABSTRACTS, vol. 105, 1986, page 82, abstract no. 35761n, Columbus, Ohio, US; N. CHINO et al.: "Structure-activity study of alpha-human atrial natriuretic polypeptide (alpha-hANP)", & PEPT.: STRUCT. FUNCT., PROC. AM. PEPT. SYMP., 9th 1985, 945-8

CHEMICAL ABSTRACTS, vol. 106, 1987, page 782, abstract no. 196787m, Columbus, Ohio, US; G. BREIPOHL et al.: "Synthesis of atriopeptin III by fragment condensation and solid phase synthesis", & KLIN. WACHENSCHR. 1986, 64(Suppl. 6), 16-20

**Description**

The present invention relates to polypeptides related to α-hANP and to their use in preparing antisera, such antisera being useful in the clinical diagnosis or in the pathophysiological research of cardiovascular disorders such as essential hypertension.

In cases of hypertension, a distinction between primary and secondary hypertension is normally made. Secondary hypertension is mainly an accompanying phenomenon to certain disorders such as renal diseases, endocrine diseases, pregnancy, aortic coarctation and central nervous disorders, and can normally be alleviated by treatment of the causative disease. Contrary thereto, primary or essential hypertension, which comprises 80—90% of all hypertension cases, is of unknown cause and can be treated by using antihypertensive medicaments, such as e.g. diuretics, vasodilators and adrenergic inhibitors.

α-hANP or α-human Atrial Natriuretic Polypeptide is the name of a polypeptide isolated from the human atrium and having a notable natriuretic and hypotensive effect. Compare Kangawa, Matsuo et al., Biochem.Biophys.Res.Comm. *118* 131 (1984) and also EP—A—0147193 cited under article 54, par. 3 EPC). This polypeptide has a molecular weight of about 3100 and is composed of 28 amino acid residues in the following sequence:

```
                    5                   10
H-Ser-Leu-Arg-Arg-Ser-Ser-Cys-Phe-Gly-Gly-Arg
                          |
                          └──────S-S ──────┐
              15                  20       |        25
Met-Asp-Arg-Ile-Gly-Ala-Gln-Ser-Gly-Leu-Gly-Cys-Asn-Ser-Phe-Arg- Tyr-OH
```

wherein Arg-II and Met-12 are interconnected through a single peptide bond.

It was reported in said article that the diuretic effect of α-hANP in biological tests using rats is about 1,500 times as potent as that of fuorsemide, thus suggesting a potential use as an antihypertensive agent, Nevertheless, it was suggested recently from another side that α-hANP is one of the substances involved in the development of cardiac diseases or essential hypertension, and this has started an extensive research on the significance of α-hANP in hypertensions.

The present inventors have searched to elucidate the pathophysiological significance of α-hANP in the living body, especially with regard to the control of water and electrolite balance. Further, they have searched to establish a specific method for the measurement of α-hANP which can be useful in the diagnosis and evaluation of cardiovascular disorders including essential hypertension.

During their research, they found that certain synthetic polypeptides which can be regarded as fragments of natural α-hANP, are capable of including antigenicity when bound to a carrier, and that such polypeptides can therefore be used with advantage for preparing an antiserum which is indispensable in establishing the just-mentioned method for measuring α-hANP in body fluids.

Therefore, the present invention provides in the first place polypeptides represented by the general formula:

R-Asn-Ser-Phe-Arg-Tyr-OH (I)

where R represents

Leu-Gly-Cys,

Gly-Leu-Gly-Cys,

Gln-Ser-Gly-Leu-Gly-Cys,

Ala-Gln-Ser-Gly-Leu-Gly-Cys,

Gly-Ala-Gln-Ser-Gly-Leu-Gly-Cys,

or Ile-Gly-Ala-Gln-Ser-Gly-Leu-Gly-Cys,

which when bound to a carrier, are capable of inducing antigenicity.

In the second place, the invention relates to the use of the just-mentioned polypeptides for preparing an antiserum useful in the clinical diagnosis or in the pathophysiological research of cardiovascular disorders.

All constituent amino acids in the general formula (I) have the L-form. The abbreviations as used are in accordance with the IUPAC—IUB nomenclature and have the following meanings:

Ala: Alanine
Arg: Arginine
Asn: Asparagine
Cys: Cysteine
Gln: Glutamine
Gly: Clycine
Ile: Isoleucine
Leu: Leucine
Phe: Phenylalanine
Ser: Serine
Tyr: Tyrosine

The polypeptides of the present invention correspond to fragments of natural α-hANP and can be indicated as such. If e.g. α-hANP itself is indicated as α-hANP-(1—28), then the polypeptides of the present invention correspond to α-hANP-(21—28), α-hANP-(20—28), α-hANP-(18—28), α-hANP-(17—28), α-hANP-(16—28) and α-hANP-(15—28).

It should be noted that some fragments of natural α-hANP, for example α-hANP-(7—28), α-hANP-(13—28) and α-hANP-(18—28) (the latter being a pyro-form peptide in which the N-terminal glutamine residue is cyclisized) are commercially available although their physiological effect and their antigen function had not yet been reported in literature.

Further, it should be noted that an octapeptide corresponding to α-hANP (21—28) and a dodecapeptide corresponding to α-hANP (17—28) were disclosed by R. F. Nutt et al in Peptides, Proc.Eur.Pept.Symp., 18th, 513—516 (1984). However, both peptides were protected peptides carrying acetamidomethyl at a cysteine residue (compare scheme I on page 515 of that article), whereas the present invention relates to unprotected polypeptides showing antigenicity when bound to a carrier. Moreover, the peptides disclosed by Nutt et al. are used only for synthetic purposes, namely for synthesizing a polypeptide of 26 amino acid units different from α-hANP, whereas the polypeptides of the present invention are used for preparing an antiserum which is useful for diagnostic purposes.

An octapeptide corresponding to α-hANP-(21—28) and a dodecapeptide corresponding to α-hANP-(17—28) are further disclosed in pages 11 and 48 and on pages 14,49 and 50 respectively of EP—A—0140731 which is cited under art. 54, par. 3 EPC. Again, these peptides are protected peptides wherein a cysteine residue is protected by acetamidomethyl.

An heptapeptide corresponding to α-hANP-(22—28) and a nonapeptide corresponding to α-hANP-(19—28) are disclosed in claim 20 and on page 5 resp. of WO—A—8502850 which is cited under article 54, par. 3 EPC. These peptides are disclaimed in the present invention.

The polypeptides of the present invention can be manufactured by conventional synthetic methods for elongation of a peptide chain, i.e. by stepwise condensation of amino acids or by coupling fragments consisting of two to several amino acids, or by a combination of both methods. A condensation between two amino acids or two peptides or between an amino acid and a peptide, can be carried out by conventional methods such as the azide method, the mixed acid anhydride method, the dicyclo hexyl carbodiimide (DCC) method, the active ester method (using a p-nitrophenyl ester, N-hydroxysuccinic acid imido ester or a cyanomethyl ester), the Woodward reagent K method, the carbonyl diimidazole method or the oxidation-reduction method. These condensation reactions may be effected either in liquid phase or in solid phase. In the case of elongating the peptide chain by a solid phase method, the peptide is attached to an insoluble carrier at the C-terminal amino acid. Useful insoluble carriers are those which react with the carboxyl group at the C-terminal amino acid to form a bond which is readily cleaved lateron, such as for Example halomethyl resins such as chloromethyl resin and bromomethyl resin, hydroxymethyl resins, aminomethyl resins, benzhydrylamine resins and t-alkyloxycarbonyl-hydrazide resins.

As usual in the synthesis of peptides, the α- and ω-side chain amino groiups and the carboxyl group of the amino acid will have to be protected and/or deprotected as far as occasion demands. Applicable amino-protecting groups are, for instance, benzyloxycarbonyl (hereinafter indicated as Z), o-chlorobenzyloxy-carbonyl [Z(2-Cl)], p-nitrobenzyloxycarbonyl [Z($NO_2$)], kunnen p-methoxybenzyloxycarbonyl [Z(OMe)], t-butoxycarbonyl (Boc), t-amyloxycarbonyl (Aoc), isobornyloxycarbonyl, adamantyloxycarbonyl, 2-(4-biphenyl)-2-propyloxycarbonyl (Bpoc), 9-fluorenylmethoxycarbonyl (Fmoc), methylsulfonylethoxy-carbonyl (Msc), trifluoroacetyl, phthalyl, formyl, 2-nitrophenylsulphenyl (NPS), diphenylphosphinothioyl (Ppt), dimethylphosphinothioyl (Mpt).

Examples of carboxyl-protecting groups are, for instance, a benzyl ester (OBzl), 4-nitrobenzyl ester [OBzl($NO_2$)], t-butyl ester (OBut) or a 4-pyridylmethyl ester (Opic). It is desirable that specific amino acids such as arginine, cysteine and serine, possessing a functional group other than amino and carboxyl groups are protected by a suitable protecting group as occasion demands. For example, the guanidino group in arginine may be protected with nitro, p-toluenesulfonyl, benzyloxycarbonyl, adamantyloxycarbonyl, p-methoxybenzenesulfonyl, 4-methoxy-2,6-dimethylbenzenesulfonyl (Mds), or 1, 3, 5-trimethylphenyl-

sulfonyl (Mts), the thiol group in cysteine may be protected with benzyl, p-methoxybenzyl, triphenylmethyl, acetylaminomethyl, ethylcarbamoyl, 4-methylbenzyl, or 2,4,6-trimethylbenzyl (Tmb), and the hydroxyl group in serine can be protected with benzyl, t-butyl, acetyl, or tetrahydropyranyl.

An example of preparing a dodecapeptide of the present invention corresponding to α-hANP-(17—28) is given below in more detail. The reaction sequence can be shown as follows.

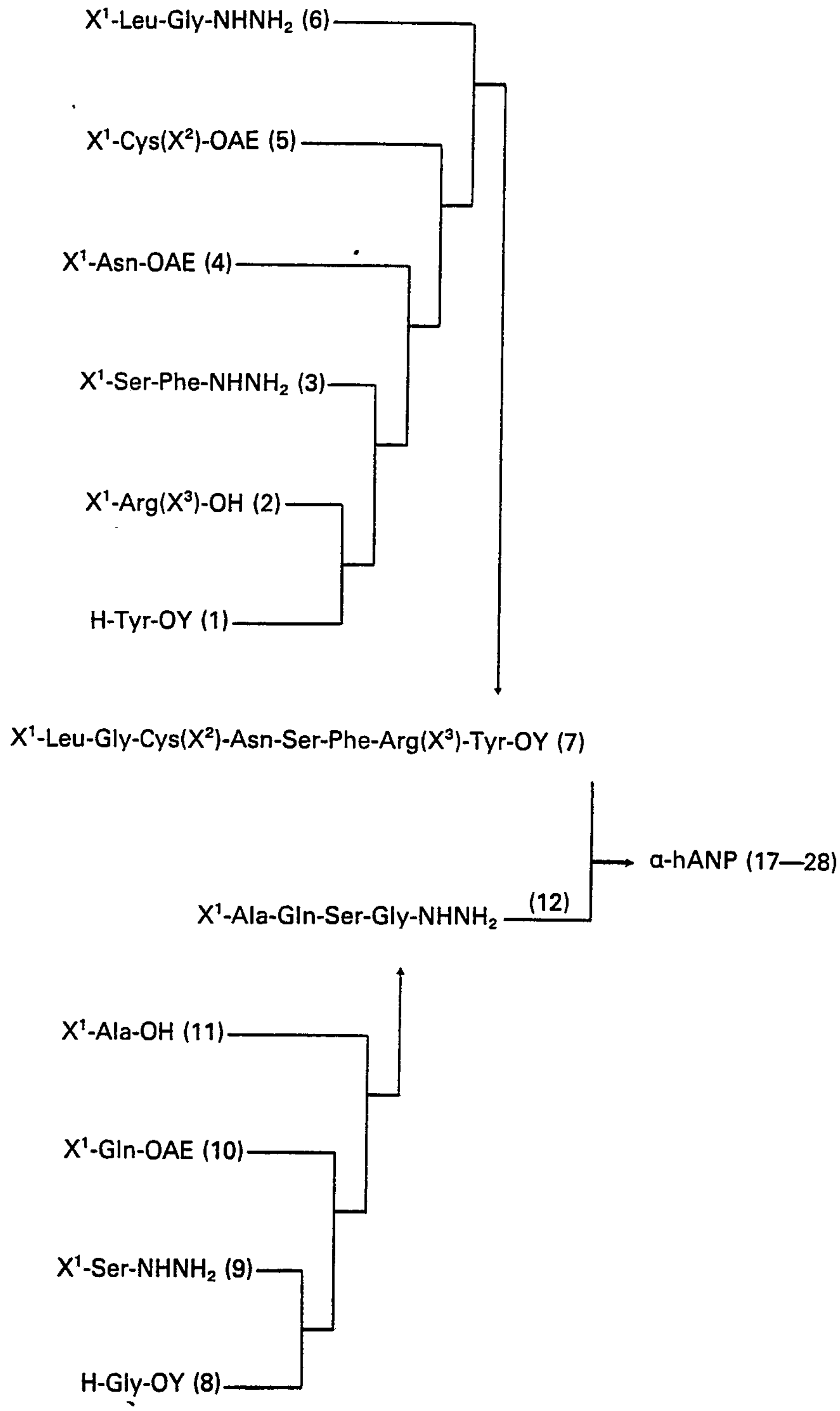


wherein

$X^1$ represents an amino protecting group for the N-terminal amino acid,

$X^2$ represents a thiol-protecting group,

$X^3$ represents a guanidyl-protecting group, AE represents an active ester residue, and Y represents a carboxyl protecting group for the C-terminal amino acid.

In the above reaction sequence favorable amino-protecting groups for $X^1$ are Z—, Z(Cl), Z(OMe), and

Boc and favorable carboxyl-protecting groups for Y are —OBzl, and OBzl($NO_2$). Tmb is a favorable thiol-protecting group for $X^2$ and Mts is a favorable guanidino protecting group for $X^3$.

In the above process, the reaction of (1) with (2), and the introduction of (11) are carried out by means of a mixed acid anhydride method. A conventional mixed acid anhydride method utilized for the synthesis of peptides is applicable here. Thus for example, arginine (2) of which the amino group and guanidino group are protected is allowed to react with the ethyl chlorocarbonate or isobutyl chlorocarbonate to give the corresponding anhydride. The anhydride-forming reaction is generally carried out under cooling or preferably at −15 to −5°C for about 10 minutes. The reaction of the anhydride with (1) is carried out in a solvent as normally used in syntheses of peptides, for example, dimethylformamide (DMF), dimethylsulfoxide (DMSO), hexamethylphosphoric triamide (HMPA), chloroform, dichloromethane, tetrahydrofuran, dioxane or ethyl acetate, under cooling (for example, ice-cooling) for 1—24 hours. The condensation reaction of (11) with the reaction product of (8), (9) and (10) to give (12) is made in the same way. In addition, the hydrazide (12) can be prepared easily from the corresponding ester on reaction with hydrazine hydrate. This hydrazide forming reaction is also utilized in the preparation of (3), (6) and (9).

A condensation reaction based on the active ester method, for example, a condensation reaction, of (4) with the reaction product of (1), (2) and (3), is attained by utilizing (4) as an active ester, e.g. as a p-nitrophenyl ester, 2,4-dinitrophenyl ester, 2,3,4,5,6-pentachlorophenyl ester, 2,4,5-trichlorophenyl ester, 2,3,4,5,6-pentafluorophenyl ester, N-hydroxysuccinate imide ester, or a homologue thereof.

The reaction can generally be carried out under cooling or heating, preferably at a temperature between −20°C and 40°C, in a solvent which can be used for the synthesis of peptides as mentioned above. The reaction time is generally 1—24 hours, though it depends on other conditions. These reaction conditions are the same in the condensation reactions of (5) and (10).

The condensation reactions of (3), (6) and (12) are achieved by the azide method. The formation of an azide from a corresponding hydrazide is achieved by means of the Curtius method (Organic Reactions Vol. 3, p. 337), i.e. a reaction with sodium nitrite in an acidic solution or the Rudinger method [Collect. Czech. Chem. Commun., *26*, 2333 (1961)] employing alkyl nitrite (for example, isoamylnitrite) in an anhydrous solvent. The reaction of the resulting azide with the corresponding peptide to be condensed is generally carried out at low temperature (from −10°C to +10°C) in the presence of a stoichiometric amount of a base. As a base, it is preferred to use organic bases, such as triethylamine, tributylamine, diisopropylethylamine, dimethylaniline, pyridine, picoline, N-methylmorpholine and the like. As a reaction solvent, those which can be used for syntheses of peptides as mentined above may be properly chosen and used. Prior to the above-mentioned reactions, it is necessary to remove the amino-protecting group represented by $X^1$. For example, a benzyloxycarbonyl-type protecting group [Z, Z(2Cl), Z($NO_2$), Z(OMe)] can be removed by treatment with hydrogen fluoride, or trifluoroacetic acid, or by catalytic hydrogenation with palladium.

The protecting groups of other functional groups which are maintained until the final step can be removed by a single or stepwise reaction in the final step. For example, Z(OMe) or Ala, Tmb of Cys, Mts of Arg, and Bzl of Tyr can be removed by a reaction with hydrogen fluoride in the presence of dimethylsulfide, as shown in the following example.

The reaction products of the various steps and the final products can be isolated and purified by conventional methods in the peptide field such as extraction, recrystallization, chromatography (gel filtration, ion exchange, partition, adsorption, or reversed phase chromatography), electrophoresis, or counter-current distribution.

The peptides of formula (I) corresponding to α-hANP-(21—20), α-hANP-(21—28), α-hANP-(18—28), α-hANP-(16—20), α-hANP-(15—28), can also be manufactured according to the above-mentioned procedure. Further, salts of such peptides can be made. These salts are exemplified by inorganic or organic acid addition salts such as hydrochlorides, hydrobromides, nitrates, sulfates, acetates, maleates, formates, lactates, tartrates, succinates and citrates, metal salts such as sodium salts, potassium salts and calcium salts, and ammonium salts or amine salts such as triethylamine salts.

Procedures for the manufacture of the objective compounds invention are given in the following examples. The present invention, is not restricted by these examples, however.

The protecting groups used in the following examples are indicated as follows:

Z(OMe): p-methoxybenxzyloxycarbonyl
OMp: p-nitrophenoxy
Bzl: benzyl
Z: benzyloxycarbonyl
Mts: 1,3,5-trimethylphenylsulfonyl (dimethylene sulfonyl)
Tmb: 2,4,6-trimethylbenzyl

Example 1

Manufacturing of a dodecapeptide corresponding to α-hANP-(17—28)

(1) Z(OMe)-Arg(Mts)-Tyr-OBzl

A solution of mixed acid anhydride, which is prepared from Z(OMe)-Arg(Mts)-OH [prepared from 10.0 g (16.1 mmol) of the cyclohexylamine salt] and isobutyl chlorocarbonate in 20 ml of dimethyl formamide (hereinafter indicated as DMF), is added to an ice-cooled solution of H-Tyr-OBzl [prepared from 8.25 g (19.3

mmol) of the corresponding toluene sulfonate] in 50 ml of DMF, and the mixture is stirred for 3 hours on an ice bath. The solvent is evaporated under reduced pressure and the oily residue dissolved in ethyl acetate, washed with 0.5 N-hydrochloric acid and with an aqueous sodium chloride solution, dried, and evaporated. The residue is crushed and pulverized, chromatographed on a column of silica gel (4.5 × 25 cm), and eluted with a chloroform/methanol (10:0.5) solvent system. The eluate is recrystallized from ethyl acetate/ether to give 11.5 g (92% yield) of the title compound. mp. 98—102°C $[\alpha]_D$ −3.2° (c=0.9, DMF, 20°C), Thin layer chromatography (TLC) on Kiesel gel 60F 245, (Merck) with a chloroform/methanol/acetic acid (9:1:0.5), as a solvent system resulted into $RF_2$ = 0.73. TLC on the same adsorbent with chloroform/methanol (10:0.5), as a solvent system resulted into $RF_3$ = 0.28. (Hereinafter the same adsorbent for TLC is used and $RF_2$ and $RF_3$ relate to the same solvent systems as indicated here).

Anal. for $C_{40}H_{47}N_5O_9S_1$
Calcd. (%): C62.08, H 6.12, N 9.05,
Found (%): C 62.19, H 6.16, N 8.66.

(2) Z(OMe)-Ser-Phe-Arg(Mts)-Tyr-OBzl

Trifluoroacetic acid (TFA)/anisole (10 ml/2.8 ml) is added to 5.0 g (6.46 mmol) of Z(OMe)-Arg(Mts)-Tyr-OBzl. The mixture is treated on an ice bath for 60 minutes, whereupon TFA is evaporated under reduced pressure. The residue is washed with n-hexane, dried on potassium hydroxide (KOH) pellets under reduced pressure for 3 hours, and then dissolved in DMF containing 0.9 ml (6.46 mmol) of triethylamine. A solution of an azide prepared from 3.34 g (7.75 mM) of Z(OMe)-Ser-Phe-$NHNH_2$ in DMF [neutralized with 2.59 ml (18.6 mM) of triethylamine] is added to the above solution under ice-cooling, and the mixture is stirred at 5°C for 14 hours, and concentrated. The residue is purified in the same extracting manner as mentioned above, then chromatographed on a column of silica gel (3.5 × 20 cm) and eluted with a solvent system of chloroform/methanol (20:0.5). The eluate is recrystallized from ethyl acetate/ether to give 4.85 g (76% yield) of the title compound. mp. 98—100°C. $[\alpha]_D$ −5.1° (c=0.6, DMF, 20°C), TLC with chloroform/methanol/water (8:3:1) as a solvent system resulted into $Rf_1$ = 0.67. (Hereinafter, the term $Rf_1$ relates to the same solvent system as indicated here).

Anal. for $C_{52}H_{61}H_7O_{12}S$
Calcd. (%): C 61.95, H 6.10, N 9.37,
Found (%): C 62.03, H 6.08, N 9.67.

(2) Z(OMe)-Asn-Ser-Phe-Arg(Mts)-Tyr-OBzl

The protected tetrapeptide ester (4.50 g; 4.46 mmol) obtained in the above procedure (2) is treated with TFA/anisole (10 ml/2.4 ml) in the same manner as mentioned above, and then dry ether is added thereto. The resulting precipitate is washed with ether, dried on KOH pellets under reduced pressure for 3 hours, and then dissolved in 20 ml of DMF. To this solution are added 0.62 ml (4.46 mmol) of triethylamine, 2.05 g (4.91 mmol) of Z(OMe)-Asn-ONp, and 0.49 ml (4.46 mmol) of N-methylmorpholine (hereinafter, indicated as NMM), whereupon the mixture is stirred for 14 hours, neutralized with acetic acid, and evaporated. The residue is crushed and pulverized in ether/water. The powder is precipitated from DMF with addition of ethanol to give 3.95 g (79% yield) of the title compound. mp. 171—173°C. $[\alpha]_D$ −15.0° (c=0.6, DMF, 20°C), TLC: $Rf_1$=0.63,

Anal. for $C_{56}H_{67}N_9O_{14}S$
Calcd. (%): C 59.93, H 6.02; N 11.23,
Found (ß): C 59.79, H 6.08, N 11.14.

(4) Z(OMe)-Cys(Tmb)-OH

A solution of 4.91 g (23.7 mmol) of Z(OMe)-$N_3$ in 25 ml of tetrahydrofuran (THF) is added under ice-cooling to a solution of 5.0 g (19.7 mM) of H-Cys(Tmb)-OH in 25 ml water containing 6.0 ml (43.3 mmol) of triethylamine. After stirring overnight at 5°C overnight, the reaction mixture is washed with ether, and then the aqueous layer is neutralized with 5% citric acid. The resulting precipitate is dried to give a powder, which is recrystallized from DMF/ether to give 4.59 g (56% yield) of the title compound. mp. 153—158°C. $[\alpha]_D$ −37.5° (c = 0.6, DMF, 20°C) TLC: $Rf_1$ = 0.56.

Anal. for $C_{22}H_{27}NO_5S$
Calcd. (%): C 63.29, H 6.52, N 3.36,
Found (%): C 63.49, H 6.54, N 3.41.

(5) Z(OMe)-Cys(Tmb)-ONp

To a solution of 5.0 g (12.0 mM) of Z(OMe)-Cys(Tmb)-OH and 1.83 g (13.2 mmol) of p-nitrophenol in 50 ml of THF is added 2.72 g (13.2 mmol) of dicyclohexylcarbodiimide (DCC). After stirring overnight at room temperature, the mixture is filtered. The filtrate is concentrated and the product is purified by crystallization from DMF/2-propanol to give 3.56 g (55% yield) of the title compound. mp. 121°C. $[\alpha]_D$ −25.5° (c = 1.0, DMF, 20°C), TLC. $Rf_5$ = 0.71 (solvent system: chloroform),

Anal. for $C_{28}H_{30}N_2O_7S$
Calcd. (%): C 62.44, H 5.61, N 5.20,
Found (%): C 62.40, H 5.61, N 5.20.

(6) Z(OMe)-Csy(Tmb)-Asn-Ser-Phe-Arg(Mts)-Tyr-OBzl

With TFA/anisole (4 ml/1 ml) is treated 2.0 g (1.78 mmol) of Z(OMe)-Asn-Ser-Phe-Arg(Mts)-Tyr-Obzl is treated with TFA/anisole (4 ml/1 ml) in a conventional way. Then dry ether is added to the mixture to give a powdery product. After drying on KOH pellets under reduced pressure for 3 hours, the powder is dissolved together with 0.23 ml (1.78 mmol) of triethylamine, 1.06 g (1.96 mmol) of Z(OMe)-Cys(Tmb)-ONp, and 0.20 ml (1.78 mM) of NNM in 20 ml of DMF. The solution is stirred for 14 hours, neutralized with acetic acid, and evaporated. The residue is crushed in water, and the resulting powder is precipitated from DMF-ethanol to give 2.01 g (83% yield) of the title compound. mp. 200—203°C. $[\alpha]_D$ −200° (c = 0.8, DMF, 20°C), TLC: $Rf_1$ = 0.51,

Anal. for $C_{59}H_{84}N_{10}O_{15}S_2$
Calcd. (%): C 61.04, H, 6.24, N 10.32,
Found (%): C 60.85, H 6.40, N 10.03.

(7) Z(OMe)-Leu-Gly-$NHNH_2$

To a solution of 7.02 g (19.7 mmol) of Z(OMe)-Leu-Gly-OMe in 30 ml of methanol is added 5.90 ml (98.5 mmol) of 80% hydrazine hydrate. The mixture is allowed to react at room temperature for 24 hours, and evaporated to dryness. Water/ether is added to the residue to crystallize. The crystals are recristallized from methanol/ether to give 5.97 g (83% yield) of the title compound. mp. 108—112°C $[\alpha]_D$ −6.0° (c = 0.7, DMF, 20°C)

Anal. for $C_{17}H_{26}N_4O_5$
Calcd. (%) C 55.72, H 7.15, N 15.29,
Found (%): C 55.74, H 7.15, N 15.20.

(8) Z(OMe)-Leu-Gly-Cys(Tmb)-Asn-Ser-Phe-Arg(Mts)-Tyr-OBzl

TFA/anisole (4.0 ml/1.0 ml) is added to 2.01 g (1.48 mmol) of Z(OMe)-Cys(Tmb)-Asn-Ser-Phe-Arg(Mts)-Tyr-OBzl and the mixture is allowed to react in a conventional manner at 0°C for 1 hour. Then TFA is evaporated, and the residue is crushed and pulverized in ether. The powder is collected by filtration, and dried to give H-Cys(Tmb)-Asn-Ser-Phe-Arg(Mts)-Tyr-OBzl·TFA. The powder is dissolved in DMF containing 0.21 ml of triethylamine. An azide solution in DMF (neutralized with 0.59 ml of triethylamine) which is prepared from 0.65 g (1.78 mmol) of Z(OMe)-Leu-Gly-$NHNH_2$ by treatment with 0.28 ml (2.14 mmol) of isoamylnitrite/3.2 M—HCl/DMF (0.28 ml/1.34 ml) is added to the above solution under ice-cooling. The mixture is stirred at 5°C for 14 hours. The reaction mixture is concentrated and water is added to the residue. The product is crystallized from DMF/ethanol to give 2.06 g (91% yield) of the title compound. mp 210—212°C. $[\alpha]_D$ −11.4° (c = 0.7, DMF, 20°C), TLC: $Rf_1$ = 0.56,

Anal. for $C_{77}H_{98}N_{12}O_{17}S_2$
Calcd. (%): C60.53, H 6.47, N 11.00,
Found (%): C 60.29, H 6.69, N 10.85.

(9) Z(OMe)-Ser-Gly-OBzl

An azide solution (neutralized with 0.26 ml of triethylamine) in 10 ml DMF is prepared from 7.0 g (24.7 mmol) of Z(OMe)-Ser$NHNH_2$ by reaction in a conventional way with 3.94 ml of isoamylnitrite/18.6 ml of 3.2 N—HCl—DMF. To this solution is added 9.51 g (29.6 mmol) of H-Gly-OBzl·toluenesulfonate (neutralized with 4.11 ml of triethylamine in 30 ml of DMF under ice-cooling and the mixture is allowed to react at 5°C for 14 hours. The reaction mixture is concentrated, and the oily residue is dissolved in ethyl acetate. The solution is washed with 0.5 N hydrochloric acid, 5% sodium hydrogen carbonate, and a saturated aqueous sodium chloride solution, dried over sodium sulfate, and evaporated to dryness. Ether is added to the residue to crystallize. The crystals are recrystallized from THF/ether to give 6.89 g (67% yield) of the title compound. mp. 95—97°C. $(\alpha]_D$ +3.0° (c = 0.7, DMf, 20PC), TLC: $Rf_1$=0.89, $Rf_3$=0.33,

Anal. for $C_{21}H_{24}N_2O_7$
Calcd. (%): C 60.57, H 5.81, N 6.73,
Found (%): C 60.67, H 5.78, N 6.84.

(10) Z(OMe)-Gln-Ser-Gly-OBzl

5.0 g (12.0 mmol) of Z(OMe)-Ser-Gly-OBzl is treated with 7FA/anisole (10 ml/2.6 ml) to remove the protecting group. The product is dissolved in 20 ml of DMF, and the solution is neutralized with 3.34 ml (24.0 mmol) of triethylamine, Then 5.69 g (13.2 mmol) of Z(OMe)-Gln-ONp is added to the solution, and the mixture is stirred for 14 hours. The solution iis concentrated, the resulting solid residue is washed with 10% citric acid and water, and recrystallized from DMF/methanol to give 5.04 g (77% yield) of the title compound. mp. 198—200°C $[\alpha]_D$ +3.9° (c = 0.5, DMF, 20°C), TLC: $Rf_1$ = 0.12,

Anal. for $C_{26}H_{32}N_4O_9$
Calcd. (%): C 57.34, H 5.92, N 10.29,
Found (%): C 57.31, H 5.98, N 10.43.

(11) Z(OMe)-Ala-Gln-Ser-Gly-OBzl

3.0 g (5.51 mmol) of the tripeptide obtained in the above procedure (10) is treated in a conventional

way with TFA (anisole (6.0 ml (1.2 ml) to remove the protecting group. The product is dissolved in 30 ml of DMF containing 0.77 ml (5.51 mmol) of triethylamine. Under ice-cooling, A solution of mixed acid anhydride (DMF 20 ml) which is prepared from 1.67 g (6.61 mmol) of Z(OMe)-Ala-OH and 0.945 ml (7.27 mmol) of isobutylchlorocarbonate is added undere ice-cooling to the above solution. The mixture is stirred on an ice bath for 3 hours. The solvent is evaporated, and the solid product which is deposited from the residue by addition of water is collected by filtration, and recrystallized from DMF/ethanol to give 2.72 g (80% yield) of the title compound. mp. 105—107°C $[\alpha]_D$ −3.6° (c = 0.8, DMF, 20°C), TLC: $Rf_1$ = 0.62

Anal. for $C_{29}H_{37}N_5O_{10}$
Calcd. (%): C 56.57, H 6.06, N 11.38,
Found (%): C 56.71, H 6.10, N 11.58.

(12) Z (OMe)-Ala)-Gln-Ser-Gly-$NHNH_2$

In 30 ml of DMF solution is dissolved 2.72 g (4.42 mmol) of the protected tetrapeptide ester prepared in the above procedure (11) is dissolved into 30 ml of DMF, whereupon 1.3 ml (5 equivalent) of 80% hydrazine hydrate is added thereto, and the mixture is allowed to react for 24 hours. The solvent is evaporated. The powdery residue is washed with ethanol, and then recrystallized from dimethyl sulfoxide (DMSO)— DMF (1:1)/ethanol to give 2.29 g (96% yield) of the title compound. mp. 216—218°C $[\alpha]_D$ −19.1° (c = 0.7, DMSO, 20°C), TLC: $Rf_1$ = 0.21. constituting amino acids (%) of the hydrolysate with 6 N—HCl: Ser 0.90, Glu 1.02, Gly 1.00, Ala 1.02. (recovery of Gly 89%).

Anal. for $C_{22}H_{33}N_7O_9$
Calcd. (%): C 48.97, H 6.17, N 18.17,
Found (%): C 48.67, H 6.38, N 18.17.

(13) Z(OMe)-Ala-Gln-Ser-Gly-Leu-Gly-Cys(Tmb)-Asn-Ser-Phe-Arg(Mts)-Tyr-OBzl [Z(OMe)-(hANP 17—28)-OBzl]

2.06 g (1.35 mmol) of Z(OMe)-Leu-Gly-Cys(Tmb)-Asn-Ser-Phe-Arg(Mts)-Tyr-OBzl is allowed to react for 60 minutes at 0°C with TFA/anisole (4 ml/1 ml). After evaporation of TFA at room temperature, dry ethanol is added to the residue to give a powdery precipitate of H-Leu-Gly-Cys(Tmb)-Asn-Ser-Phe-Arg(Mts)-Tyr-OBzl·TFA. The product is collected by filtration, dried on KOH pellets for 3 hours under reduced pressure, and then dissolved in 10 ml of DMF containing 0.19 ml (1.35 mmol) of triethylamine.

On the other hand, 1.09 g (2.03 mmol) of Z(OMe)-Ala-Gln-Ser-Gly-$NHNH_2$ is dissolved into 10 ml of DMF—DMSO (1:1), and 1.22 ml (4.87 mmol) of 4.0 N-hydrochloric acid/DMF and 0.32 ml (2.44 mmol) of isoamylnitrite are successively added to the solution. The mixture is allowed to react under cooling, and neutralized with 0.68 ml (4.87 mmol) of triethylamine to give a solution of the corresponding azide. This azide solution is dropwise added to the above solution of octapeptide of DMF under ice-cooling, and the mixture is stirred at 5°C overnight. After confirmation of a negative ninhydrin reaction, the reaction mixture is diluted with 50 ml of water. The resulting powdery precipitate is collected by filtration, dried, and recrystallized from DMF/90% methanol to give 2.37 g (94% yield) of the title compound. mp. 242—244°C, $[\alpha]_D$ −7.1° (c = 0.6, DMSO, 20°C), TLC: $Rf_4$ = 0.85 solvent system: n-butanol/pyridine/acetic acid/water (4:1:1:2)].

Constituting amino acids (%) of the hydrolysate with 6 N—HCl: Asp 1.03, Ser 1.90, Glu 1.21, Gly 2.17, Ala 1.17, Cys 0.66, Leu 0.98, Tyr 0.97, Phe 1.00, Art 1.03. (recovery of Phe 97%)

Anal. for $C_{90}H_{119}N_{17}O_{23}S_2$
Calcd. (%): C 57.77, H 6.41, N 12.73,
Found (%): C 57.56, H 6.48, N 12.51.

(14) H-Ala-Gln-Ser-Gly-Leu-Gly-Cys(SH)-Asn-Ser-Phe-Arg-Tyr-OH [α]-hANP-(17—28)].

To 100 mg (53.4 μ mol) of Z(OMe)-[α-hANP-(17—28)]-OBzl obtained in the above procedure (13) are added 280 μl (2.67 μmol) of m-cresol, 196 μl (2.67 μmol) of dimethyl sulfide, and 2 ml of hydrogen fluoride. The mixture is kept at 0°C for 1 hour, whereupon hydrogen fluoride is evaporated at 0°C. Ether is added to the residue, and the resulting powdery substance is centrifuged, dissolved into 2 ml of water containing 82 mg (10 equivalent) of dithiothreitol, and adjusted to pH 8 with addition of 3% aqueous ammonia. The mixture is stirred for 30 minutes under flowing argon. The reaction mixture is purified on Sephadex G—25 (Pharmacia AB) (1.8 × 140 cm) and eluted with 0.2 N acetic acid (the volume of each fraction is 7 ml.). The eluate is freeze-dried to give 23 mg (33% yield) of the title compound. $[\alpha]_D$ −10.0° (c = 0.1, 0.2 N acetic acid, 30°C), TLC: Rf = 0.37. Constituting amino acids (%) of the hydrolysate with 6 N—HCl: Asp 0.98, Ser 1.85, Glu 1.03, Gly 2.02, Ala 1.09, CySH 1.36, Leu 0.99, Tyr 0.78, Phe 1.00, Arg 0.97. (recovery of Phe 92%).

## Example 2

Manufacture of α-hANP-(24—28).

To 93 mg (0.0829 mmol) of Z(OMe)-Asn-Ser-Phe-Arg(Mts)-Tyr-OBzl, the protected pentapeptide obtained in Example 1—(3), are added 87 μl (0.829 mmol) of m-cresol, 59 μl (0.829 mmol) of dimethyl sulfide, and 2 ml of hydrogen fluoride. The mixture is allowed to react at 0°C for 1 hour whereupon hydrogen fluoride is evaporated at 0°C, and the residue is worked up in the same manner as in Example 1-(14) to give 34 mg of α-hANP-(24—28) [H-Asn-Ser-Phe-Arg-Tyr-OH].

Example 3

Manufacture of H-Ile-Gly-Ala-Gln-Ser-Gly-Leu-Gly-Cys(SH)-Asn-Ser-Phe-Arg-Tyr-OH corresponding to α-hANP-(15—28).

500 mg (0.267 mmol) of Z(OMe)-Ala-Gln-Ser-Leu-Gly-Cys(Tmb)-Asn-Ser-Phe-Arg(Mts)-Tyr-Obzl is allowed to react at 0°C for 1 hour. with 0.23 ml (2.14 mmol) of anisole and 2 ml of TFA. Then, TFA is evaporated at room temperature, either is added to the residue to pulverize, and the powder is collected by filtration and dried to give the deprotected product as a trifluoroacetate from which Z(OMe) has been removed, i.e. H-Ala-Gln-Ser-Gly-Leu-Gly-Cys(Tmb)-Asn-Ser-Phe-Arg(Mts)-Tyr-OBzl·TFA.

Separately, 127 mg of Z(OMe)-Ile-Gly-$NHNH_2$ is allowed to react with 5 ml of DMF, 208 μl of 4.0N—HCl/DMF, 55 μl of isoamylnitrite, and 116 μl of triethylamine to give the corresponding azide. The azide is dropwise added to the above deprotected product in DMSO—DMF (1:1) under ice-cooling, and the mixture is allowed to stand overnight under stirring. The solid matter which is produced from the reaction mixture with addition of water is collected by filtration, dried, and further purified by reprecipitation from DMF/methanol to give 433 mg (79.4% yield) of the desired protected α-hANP-(15—28), that is, Z(OMe)-Ile-Gly-Ala-Gln-Ser-Gly-Leu-Gly-Cys(Tmb)-Asn-Ser-Phe-Arg(Mts)-Tyr-OBzl. TLC: $Rf_1$ = 0.47.

Constituting amino acids (%) of the hydrolysate with 6 N—HCl: Asp 1.00(1), Ser 1.83(2), Gln 1.21(1), Cystein 0.60(1), Gly 3.46(3), Ala 1.24(1), Ile 1.07(1), Leu 1.20(1), Tyr 0.92(1), Phe 1.00(1), Arg 0.92(1). (recovery of Phe 63%).

The above protected α-hANP-(15—28) (100 mg; 0.049 mmol) is treated with 180 μl (2.45 mmol) of methyl sulfide and 2 ml of hydrogen fluoride in 257 μl (2.47 mmol) of m-cresol at 0°C for 1 hour. Thereafter, hydrogen fluoride is evaporated under reduced pressure at 0°C. The residue is pulverized with addition of ether and the powder is collected by centrifugation. The product is dissolved in 2 ml of aqueous solution containing 75 mg of dithiothreitol. The mixture is adjusted to pH 8 with addition of 5% aqueous ammonia, and stirred under flowing argon for 30 minutes. The reaction product is chromatographed on a column of Sephadex G—25 (1.8 × 110 cm) (pharmacia AB), and eluted with 0.2 N acetic acid (volume of each fraction, 6 ml). The fractions 30 to 37 are freeze-dried to give 58 mg of the title compound, α-hANP-(15—28), in 80% yield. TLC: $Rf_4$ = 0.29.

It has been confirmed as shown in the following experiment that the polypeptides of the present invention, when bound to a carrier, are capable of inducing antigenicity in animals just like α-hANP-(1—28) and that an antiserum prepared from these polypeptides can be utilized in a radioimmunoassay specific to α-hANP in order to elucidate a pathophysiological role of α-hANP in a capacity-controlling system.

Experiment 1

Combination of α-hANP-(17—28) with bovine tyroglobulin. (Preparation of a peptide-tyroglobulin complex).

α-hANP-(17—28) is combined with bovine tyroglobulin by the carbodiimido-coupling method [Yoshimasa et al., J. Clin. Invest. *69*, 643 (1983), Nakao et al., Biochem. Biophys. Res. Commun. *117*, 695 (1984)].

3 mg of α-hANP-(17—28) and 25.2 mg of bovine tyroglobulin, were dissolved into 2 ml of distilled water and a solution of 30 mg of 1-ethyl-3-(3-dimethylaminopropyl)carbodiimide hydrochloride was added thereto. The mixture was adjusted to pH 5.6 with addition of hydrochloric acid, and stirred at room temperature for 20 hours. The reaction mixture was dialyzed 3 times at 4°C against 5 l of distilled water to give the complex.

Immunization

100—200 μg of α-hANP-(17—28)-tyroglubulin complex, was suspended into an equal volume of Freud's complete adjuvant.

Japanese white rabbits wer immunized with the suspension by subcutaneous injection to their interscapular vertebrae area. Every 4 weeks, a booster injection was given, and 10—14 days after each booster injection the blood was collected to give antiserum $CR_3$.

Iodination

1 μ g of α-hANP-(1—28) was iodinated with 1 mCi $^{125}I_2$ in a usual manner according to the chloramin T method [Nature, *194* 495 (1962)]. The reaction mixture was passed through a Sep-Pak cartridge (Waters Ltd.) and eluted with a 50% acetonitril/5 mM trifluoroacetic acid mixture to give a pure product. The specific acitivity of the $^{125}I$-labelled α-hANP was 700 μCi/μg.

Radioimmunoassay (RIA)

A 0.1 M phosphate buffer containing 0.5% gelatin, 0.1 mM EDTA-2Na, 0.2 mM cystine, 0.1% Triton X—100 and 0.01% merthiolate was used as a buffer solution for RIA.

To each 100 μl of a standard solution of α-hANP were added 100 μl of antiserum $CR_3$ solution (4000-fold final dilution), 100 μl of $^{125}$—α-hANP (about 5,000 cpm), and 200 μl of the buffer solution for RIA. The mixture was incubated at 4°C for 72 hours.

A dextran-coated charcoal suspension (1 ml) consisting of 300 mg/100 ml of charcoal (Norit SX Plus), 30 mg/100 ml of dextran (Dextran T—70, Pharmacia AB), and 1 ml of 0.05 M phosphate buffer containing

0.01% merthiolate was added thereto. The mixture was allowed to stand at 4°C for 15 minutes, and thereupon the conjugate of $^{125}I$ labelled α-hANP was separated by centrifugation from the free form thereof, and the radio activity was measured.

Results

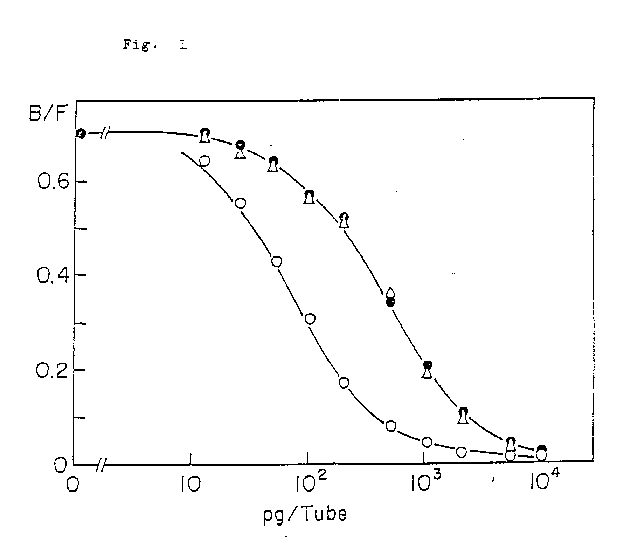

The results of the experiment are graphically represented in fig. 1 of the drawing where the ratio of bound antigen (B) to free antigen (F) is given on the vertical axis and the concentration of α-hANP (pg/tube) is given on the horizontal axis. Thus, fig. 1 shows a standard curve for α-hANP-(1—28) represented by ●—●.

Other experiments were made with α-rANP-(1—28) isolated from the rat atrium and with a synthetic peptide corresponding to α-hANP-(24—28). The results are also represented in fig. 1, thus showing a cross-curve for α-rANP-(1—28) represented by Δ—Δ, and a cross curve for α-hANP-(24—28) represented by ○—○.

In these experiments, the minimum detection limit was 50 pg.

Conclusion

It is clear from fig. 1 that the antiserum $CR_3$ prepared from α-hANP-(17—28) recognizes α-hANP-(1—28) and α-rANP-(1—28) equally. On the other hand, it is confirmed from molecular weight conversions that α-hANP-(24—28) has an activity which is equivalent to that of α-hANP-(1—28).

The radio immuno assay could detect α-ANP—LI in extracts from dog, guinea pig, monkey, bovine, porcine, and sheep atria as expected. This result indicates that the RIA system as shown above is applicable not only to the detection of α-hANP but also to the detection of α-ANP related peptides from other animal species. The system is also applicable to the detection of other natural or synthetic α-ANP like peptides of which the amino acid sequence has not yet been determined.

**Claims**

1. A polypeptide represented by the general formula:
R-Asn-Ser-Phe-Arg-Tyr-OH
wherein R represents

Leu-Gly-Cys,

Gly-Leu-Gly-Cys,

Gln-Ser-Gly-Leu-Gly-Cys,

Ala-Gln-Ser-Gly-Leu-Gly-Cys,

Gly-Ala-Gln-Ser-Gly-Leu-Gly-Cys,

or Ile-Gly-Ala-Gln-Ser-Gly-Leu-Gly-Cys,

which, when bound to a carrier, is capable of inducing antigenicity.

2. A polypeptide as claimed in claim 1, wherein R is Ala-Gln-Ser-Gly-Leu-Gly-Cys.

3. A polypeptide as claimed in claim 1, wherein R is Ile-Gly-Ala-Gln-Ser-Gly-Leu-Gly-Cys.

4. use of the polypeptide of any of claims 1—3, for preparing an antiserum useful in the clinical diagnosis or in the pathphysiological research of cardiovascular disorders.

**Patentansprüche**

1. Polypeptid, dadurch gekennzeichnet, daß es durch die allgemeine Formel: R-Asn-Ser-Phe-Arg-Tyr-OH dargestellt ist, worin R für:

Leu-Gly-Cys,

Gly-Leu-Gly-Cys,

Gln-Ser-Gly-Leu-Gly-Cys,

Ala-Gln-Ser-Gly-Leu-Gly-Cys,

Gly-Ala-Gln-Ser-Gly-Leu-Gly-Cys,

oder Ile-Gly-Ala-Gln-Ser-Gly-Leu-Gly-Cys,

steht, das bei Bindung an einen Träger in der Lage ist, Antigenität zu induzieren.

2. Polypeptid nach Anspruch 1, dadurch gekennzeichnet, daß R für Ala-Gln-Ser-Gly-Leu-Gly-Cys steht.

3. Polypeptid nach Anspruch 1, dadurch gekennzeichnet, daß R für Ile-Gly-Ala-Gln-Ser-Gly-Leu-Gly-Cys steht.

4. Verwendung des Polypeptids nach einem der Ansprüche 1 bis 3 zur Herstellung eines Antiserums, das zur klinischen Diagnose oder zur pathophysiologischen Erforschung kardiovasculärer Störungen geeignet ist.

**Revendications**

1. Polypeptide représenté par la formule générale:

R-Asn-Ser-Phe-Arg-Tyr-OH

dans laquelle R représente:

Leu-Gly-Cys,

Gly-Leu-Gly-Cys,

Gln-Ser-Gly-Leu-Gly-Cys,

Ala-Gln-Ser-Gly-Leu-Gly-Cys,

Gly-Ala-Gln-Ser-Gly-Leu-Gly-Cys,

ou Ile-Gly-Ala-Gln-Ser-Gly-Leu-Gly-Cys,

qui, lorsqu'il est lié à un support, est capable d'induire une antigénicité.

2. Polypeptide selon la revendication 1, dans lequel R représente Als-Gln-Ser-Gly-Leu-Gly-Cys.

3. Polypeptide selon la revendication 1, dans lequel R représente Ile-Gly-Ala-Gln-Ser-Gly-Leu-Gly-Cys.

4. Utilisation du polypeptide selon l'une des revendications 1—3, pour la préparation d'un antisérum utile dans le diagnostic clinique ou dans la recherche pathophysioligique des troubles cardio-vasculaires.

Fig. 1